# EUROPEAN PATENT SPECIFICATION

(11) **EP 0 909 388 B1**
(45) Date of publication and mention of the grant of the patent: **24.09.2003**
(21) Application number: 98903272.7
(22) Date of filing: 06.02.1998
(51) Int. Cl.: G01N 33/68, G01N 33/566, C07K 16/18, C07K 14/47

(54) **IMMUNOLOGICAL ASSAY FOR SPONGIFORM ENCEPHALOPATHIES**
IMMUNOASSAY FÜR SPONGIFORME ENCEPHALOPATHIEN
DOSAGE IMMUNOLOGIQUE DESTINE AUX ENCEPHALOPATHIES SPONGIFORMES

(30) Priority: 06.02.1997 IE 970081; 24.03.1997 IE 970228; 01.05.1997 IE 970325
(43) Date of publication of application: 21.04.1999
(73) Proprietor: ENFER TECHNOLOGY LIMITED, Dublin 2 (IE)
(72) Inventor: O'CONNOR, Michael, Mount Merrion, Co. Dublin (IE)
(74) Representative: Gates, Marie Christina Esther
(86) International application number: IE9800007
(87) International publication number: WO98035236

(56) References cited:
- WO-A-93/11155
- WO-A-97/10505
- WO-A-97/37227
- US-A- 4 806 627

## Description

The present invention relates to method of detecting Transmissable Spongiform Encephalopathies and to an immunological assay or test for Transmissable Spongiform Encephalopathies (TSE).

### Background to the invention

Spongiform Encephalopathies are a group of degenerative neurological diseases. There are a number of examples of Spongiform Encephalopathies including BSE (Bovine Spongiform Encephalopathy), Scrapie, Creutzfeldt-Jakob Disease (CJD), Gerstmann-Straussler-Scheinker Syndrome, Kuru, Transmissable Mink Encephalopathy, Chronic Wasting Disease of Mule Deer, Feline Spongiform Encephalopathies and other Spongiform Encephalopathies found in animals such as elk, nyala, greater kudu, gemsbok and tigers. It has also been reported that BSE can be transmitted under laboratory conditions to mice and pigs. This crossing of species barriers by the infective agent has led to increased concern that transfer to humans could occur.

Bovine Spongiform Encephalopathy (BSE) is a degenerative brain disorder of cattle which is popularly known as "mad cow disease". It has a slow incubation period, up to four or five years with symptoms of progressive degeneration of the mental state in cows include loss of coordination and staggering gait, lack of interest in their surroundings, disinterest in feed and water, or unpredictable behaviour, including aggressiveness. Affected cattle show symptoms when they are three to ten years old.

First identified in Great Britain in November 1986, over 100,000 cases have since been recorded there. Post mortems of affected cattle reveal a characteristic pattern of vacuolation in the brain tissue due to destruction of neural cells and the deposition of unusual protein fibres, that give the brain a spongy (spongiform) texture. Similar spongiform diseases have been recognized in humans (for example, Creutzfeldt-Jakob disease or CJD) for over a century and in sheep (scrapie) for over 200 years. The agent thought to be responsible for BSE and its counterparts is an infective protein known as a prion. The prion is an infective particle comprising protein only and no nucleic acid, the presence of nucleic acid being required in the case of a conventional virus. In Scrapie in particular one protein known as prion protein or PrP^{SC}, has been found to co-purify with infectivity and can produce a Scrapie-like condition in brain cell cultures from other animals, such as hamsters under laboratory conditions. PrP^{SC} is the only known component of the characteristic protein fibres deposited in the brain tissue of Scrapie-infected sheep. This protein, the PrP^{SC} appears to undergo a structural modification, whereas the term PrP^{C} is used in respect of the normal cellular counterpart of PrP^{SC}. The natural function of PrP^{C} is not known, but it appears likely that it has an essential structure or functional role in the organism.

Recycled animal tissue, which had been routinely fed to British dairy cows as a protein supplement has been identified as the source of the infection. It is believed that BSE was originally spread from sheep's brains infected with scrapie and that its spread was accidentally accelerated by the ingestion of brain tissue taken from cows that had become infected with BSE. Therefore, the British Government introduced compulsory destruction of suspect animals and their carcasses beginning in 1988. The feeding of animal tissue to cows was banned in Britain in July 1988.

Since the initial report of the disease, consumers have feared that it might be transferable to humans through milk or beef products, particularly since Kuru, a related disease is known to be spread by ritualistic cannibalism among New Guinea tribesmen. In late 1990, consumer concern over the transmission of BSE to humans triggered a collapse in the beef market. A similar scare struck Germany in mid-1994.

In 1996 ten cases of a newly described type of fatal CJD (Variant CJD) were identified. The victims had distinct brain tissue symptoms, were all under the age of 42, and had no hereditary record of the disease. It has been suggested that the victims may have contracted the disease through contact with BSE-infected cattle before the eradication of suspected animals had taken effect. The identification of variant CJD led to a dramatic drop in beef consumption in Britain, and the banning of British and in some instances Irish beef imports in various countries worldwide.

Therefore, for both veterinary and economic reasons, there is an urgent need to provide a method for diagnosis and a diagnostic kit to detect infection with BSE, Scrapie and other related Spongiform Encephalopathies, in livestock, animal carcasses and meat generally.

WO-A-93/11155 of Proteus Molecular Design Ltd. discloses methods of raising antibodies against a synthetic peptide sequence and the use of the antibodies in immunoassays that detect encephalopathies.

US-A-4,806,627 discloses a monoclonal antibody capable of binding into scrapie-associated proteins in an antigen-antibody complex.

WO-A-97/37227 teaches a method of diagnosing encephalopathies using polypeptide sequences from prion proteins obtained from sheep affected by scrapie to generate antisera specific for those proteins.

WO-A-97/10505 teaches an assay for the detection of rogue prion proteins using an antibody which binds to prion associated protein.

### Object of the invention

It is therefore an object of the present invention to provide a method of testing cattle, particularly animal carcasses, for the infective agent responsible for BSE. It is also an object that protection method be rapid, with the result being available in a matter of hours, that it should be cheap, reliable and user-friendly.

Such a detection method would have the advantage that it would prevent the entry of infected meat into the human food chain, thus eliminating the possibility of humans contracting Variant CJD or other related diseases which may be transmitted by eating infected meat. It has the further advantage that it would restore consumer confidence in meat and meat products, which would be advantageous for both the farming community and the meat industry in general.

At present there is no test available which can identify infected meat carcasses or livestock carcasses, and there is no product available which can allay public fears regarding meat consumption.

The current invention provides an immunological assay for the putative agent PrP^{SC} the rogue prion protein believed to be responsible for Spongiform Encephalopathies.

### Summary of the invention

According to the present invention there is provided a method "in vitro" for detecting the putative agent for TSE in animals comprising reacting a body tissue sample taken from an animal in a immunological assay with a labelled antibody which is capable of reacting with PrP^{SC} and determining the amount of labelled antibody bound to the sampled, wherein the antibody is a prion specific antibody raised against one or more of the following sequences:

MVKSHIGSWILVLFVVAMWSDVGLCKKRPKPGGGWNTGGSRYPGQ-44
GSPGGNRYPPQGGGGWGQPHGGGWGQPHGGGWGQPHGGGQGQP-87
GGGGWGQGGSHSQWNKPSKPPKTNMKHVAGAAAGAVVGGLGGY-131
MLGSAMSSPLIHFGNDYEDRYTRENMYRYPNQVYYRPVDRYSNQNN-177

The antibody used in the assay is preferably a C5 antibody to PrP^{SC} which is available from Proteus, England, called herein the differentiation reagent.

In a preferred embodiment, the immunological assay is a competitive assay in which a solid support, suitably a microtitre plate, is pre-coated with a carrier protein-peptide conjugate, the animal tissue sample and an anti-peptide antibody are added to the solid support, allowed to react and the support washed, a labelled anti-(anti-peptide antibody) antibody is added, allowed to react, washed, a signal reagent added and the signal read. The label may be horseradish peroxidase.

The primary antibody used in the assay is preferably a rabbit anti-P^{r}P^{SC} antibody and the secondary antibody is preferably selected from a goat, sheep, donkey or other anti-rabbit antibody.

In a particular embodiment an ELISA assay can be used to identify the peptide fragment for PrP^{SC}, but other suitable immunological assays could be used.

In a preferred embodiment an enhanced chemiluminescence assay is used to aid in detection of the PrP^{SC} agent. The animals may suitably be cattle, sheep and pigs and the tissue sample may suitably be taken from a carcass of such animals.

The steps involved are:
i. Clean up of nervous tissue to allow detection of putative agent for PrP^{SC} (the rogue prion protein believed responsible for Spongiform Encephalopathies);
2. Assay priming step involving addition of specific agents (antibodies);
3. ELISA using wells pre-coated with peptide fragment for PrP^{SC};
4. Enhanced chemiluminescence assay for detection of PrP^{SC} agent; and
5. Determination of results.

In particular the assay involves assay for the PrP^{SC} protein which is the infective prion protein found in BSE. The assay is capable of distinguishing between PrP^{SC} and PrP^{SC} which is the normal BSE prion protein. The PrP^{SC} protein is a series of peptides in a triple helix whereas in PrP^{SC} one third of the molecule is in the form of a flat beta-sheet.

It is particularly important that the assay can discriminate between natural PrP^{C} and PrP^{SC}, since PrP^{C} is found in normal subjects while both PrP^{C} and PrP^{SC} are found in diseased subjects.

In a particularly preferred embodiment, a sample of CNS tissue, suitably a cross-section of spinal cord, is removed from an animal carcass, homogenised, filtered and plated onto a microtitre tray. The sample is then reacted in an immunological assay with an antibody as described above.

The invention also provides a test kit for the detection of TSE in animals comprising an anti-peptide antibody as defined above.

The method allows the rapid detection of a TSE agent in a carcass, with results being available in a matter of hours, usually about one and a half to two hours. Thus the carcass can be removed from the abattoir before passing into the human food chain.

### Detailed description of the invention

The invention will now be described in greater detail with reference to the Examples.

The reguirements for the Enfer TSE immunoassay are as follows:
a) A sample of CNS tissue
b) A series of preparative sample treatments
c) A prion specific antibody
d) Sensitive method of detection

a) A sample of CNS tissue is removed from a beef carcass at the point of slaughter using a specialised sampling clavical (available from Medisteel, Dublin, Ireland). This tissue sample must be such that a cross-section of spinal cord can be removed from the sample for confirmatory analysis if necessary. The sample is placed in a universal container and is identified with a traceable identification number i.e. an EU 4 digit carcass number, an Irish Department of Agriculture ear-tag number or a traceable factory kill number. The sample is transported to the laboratory for testing.
b) On reaching the laboratory the sample is identified using barcodes. The barcode assigned to each sample incorporates information on the factory of origin of the sample, the date the animal is killed and a traceable identification sample number. An amount of each sample, ranging in weight from 0.3g to 1.1g, is removed and placed into a stomacher bag for homogenisation. This bag containing a section of the original sample will be assigned an identical barcode to the original sample. This allows full traceability throughout the system. A fixed volume of Homogenising Buffer is added to the stomacher bag and the sample homogenised. The sample is then dispensed in duplicate onto a prepared 96 well microtitre plate. A fixed volume of Differential Buffer is applied to the microtitre plate and incubated. A fixed volume of Priming Buffer is then added to the samples on the plate and incubated. Tills step completes the sample preparation procedure.
c) The samples are now ready for immunoassay. This requires a prion specific antibody. This antibody is raised in rabbits to a synthetic prion peptide. A fixed volume of this specific antibody is added to the microtitre plate, incubated and washed. A fixed volume of secondary antibody is added to the plate, incubated and washed. Detection of results is now possible.
d) Detection of results is by means of Enhanced Chemiluminescence. A fixed volume of a chemiluminescence reagent is added to the microtitre plate and incubated. The light signal is read using a Labsystems Chemiluminometer, the results assigned to the corresponding barcode and a results report printed.

### Example 1

### REAGENTS

### REAGENTS FOR HOMOGENISATION OF SAMPLES

Enfer Homogenisation Buffer (HB)
Reverse Osmosis Water
Differentiation Reagent
Positive Control
Negative Control
Enfer Immunoassay Priming Buffer (IPB)

### REAGENTS FOR THE IMMUNOASSAY PROCEDURE

1.5 M Phosphate Buffered Saline (PBS)
150mM Phosphate Buffered Saline (PBS)/Tween 20 (0.05%)
Sodium Chloride Solution (NaCl)
Rabbit Anti-PrP peptide in 150 mM PBS/Tween 20 (0.05%) diluted as instructed by the supplier
Normal Goat Serum
Donkey Anti-Rabbit IgG-Horse Radish Peroxidase conjugate in 150mM PBS/Tween 20 (0.05%) diluted as instructed by the supplier Amerlite Reagent ^{Tm} Johnson & Johnson Clinical diagnostics

### EQUIPMENT

Bar-code reader and computer database interface
Bar-code label printer
Rotating (bottle) mixer
96 well microtitration plate chemiluminescence reader
96 well microtitration plate shaking incubator (2)
96 well microtitration plate washer (2)
Micro-computer
Laser printer

Micro-computer 'custom' software - data processing and reporting Deep Freeze Storage -20°C
Refrigerated Storage 4°C
Reverse Osmosis Water System
Automated 8 channel pipettes
Automated microtitre plate strip dispenser
Vortex Mixer
Disposable Pasteur pipettes
Stomacher homogeniser and bags (variable quantity)
Blades
Secure sample boxes and transport trailers
Class II Safety Cabinet system
Autoclave

### SAMPLES AND SAMPLING PROCEDURE

The sample shall be such as to enable the detection of PrP^{SC} protein in spinal chord.
The size of the sample will be sufficient to permit the primary analysis and if necessary, a repeat or confirmatory analysis to be carried out.
Samples will be taken in a manner which will ensure tnat a fixed amount of tissue will be taken for each sample.
Samples will be taken in a manner which will permit identification of the sample in the laboratory.
The method of packaging, preservation and transport to the laboratory will maintain the integrity of the sample such that the results of the analysis is not prejudiced.
Samples for analysis will be transported to the laboratory, in insulated and sealed containers.

### PROCEDURE

### HOMOGENISATION AND PREPARATION OF TISSUE

7.5 ml Homogenisation Buffer (HB) is added to each sample.
The sample is sealed and placed in the homogeniser, homogenise for 3 minutes.
Remove the sample from the homogeniser and after accumulation of 40 samples, place the samples onto a designated rack.
Apply 0.02ml differentiation reagent to each well of the pre-coated microtitre plate except wells A1,A2.
Transfer, in order, the identification bar-code of each sample to the microcomputer with a bar-code scanner.
Apply 0.18ml of sample to each well except A1, A2, A3, A4 (controls).
Cover the plate with a microtitre plate sealer.
Incubate the microtitre plate for 1 hour at 37°C, shaking.
Wash the microtitre plate wells 8x with 0.4ml NaCl solution.
Dispense 0.25ml of the Immunoassay Priming Buffer to each well. Incubate the microtitre plate for 15 minutes at 37°C, shaking.
Wash the microtitre plate wells 4x with 0.4ml PBS/Tween 20 (0.05%) solution.

### CHEMILUMINESCENT IMMUNOASSAY

Dispense 0.2ml primary antibody into each well of the microtitration plate.
Incubate the microtitration plate for 40 minutes, with shaking, at 37°C.
Wash the microtitration plate wells 4x with 0.4ml of PBS/Tween 20 (0.05%) solution.
Dispense 0.2ml secondary antibody into each well of the microtitration plate.
Incubate the microtitration plate for 30 minutes, with shaking, at 37°C.
Wash the microtitration plate wells 4x with 0.4ml of PBS/Tween (0.05%) solution.
Dispense 0.15ml Amerlite ^{Tm} reagent into each well of the microtitration plate.
Incubate the microtitration plate for 3 minutes, with shaking, at 37°C.
Read the luminescence in the reader.
Data reduction and interpretation.

### CALCULATION OF RESULTS

### N/A

### ANTIBODIES, HOMOGENISATION BUFFER, IMMUNOASSAY PRIMING BUFFER, QUALITY CONTROLS

### ANTIBODIES

* rabbit Anti-PrP peptide, stored frozen and diluted to working strength as instructed by the supplier.
* donkey Anti-Rabbit IgG-Horse Radish Peroxidase (HRP) conjugate, stored at 4°C, to be diluted as instructed by the supplier.
* normal goat serum, stored at 4°C, to be diluted as appropriate.

### QUALITY CONTROLS

Supplied by Veterinary Research Laboratories, Abbotstown, Dublin.

### SOURCE OF ALL REAGENTS

Enfer Scientific Ltd.
Co. Tipperary.
Ireland.

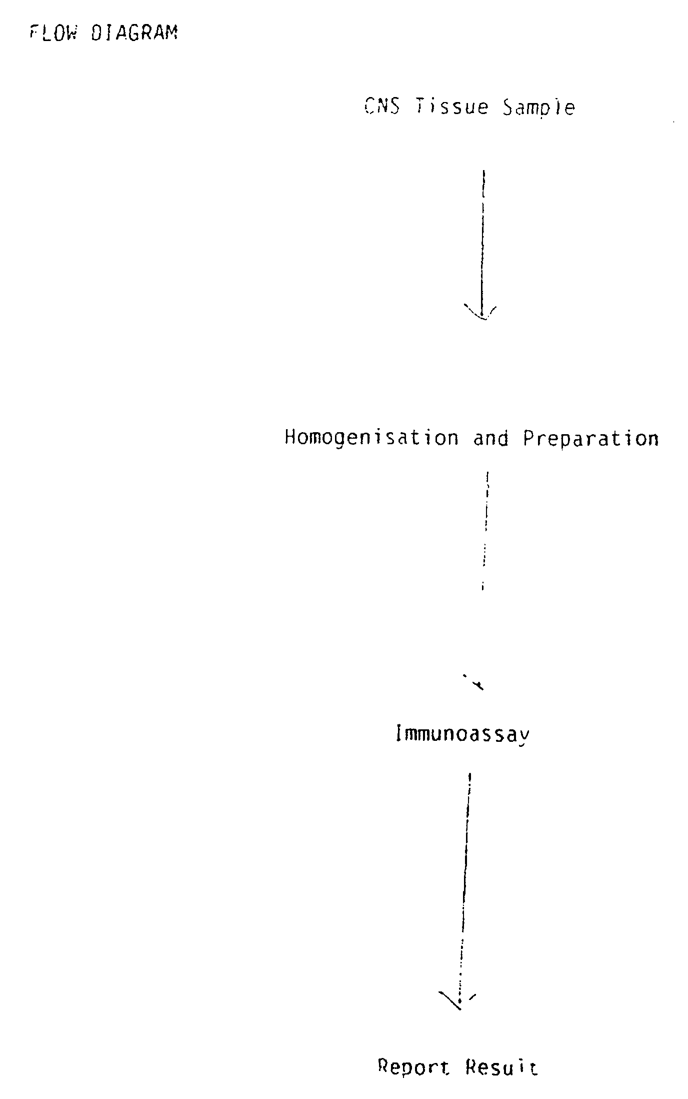

### Example 2

### 1. Homogenisation:

1.1 1g of CNS tissue is removed from the sample and placed in a stomacher bag.
1.2 15ml of Homogenising Buffer is added to the section.
1.3 This mixture is homogenised for 3 minutes using a stomacher homogeniser.
1.4 The resultant homogenate is filtered through a 1 micron filter.

### 2. Plating:

2.1 96-well microtite plate is prepared by dispensing 200 ul of Adhering Agent into each well and incubating overnight at 37°C.
2.2 Wash the plate 4XPBST (5 x Phosphate Buffered Saline tablets, supplied by Sigma, U.K., to 1 litre reverse osmosis H₂O/1% Tween 20) (150mM) before use.
2.3 200ul of blank control (such as water, saline solution or buffer) is dispensed in duplicate onto the plate, positions A1,2.
2.4 200ul of negative control (known negative BSE homogenate) is dispensed - 4 replicates - onto the plate, positions B1,2 C1.2. The known negative BSE homogenate is supplied by the Veterinary Research Laboratory, Abbotstown, Castleknock, Dublin, Ireland.
2.5 200ul of positive control (known positive BSE homogenate, available from the Veterinary Research Laboratory, Abbotstown, Castleknock, Dublin, Ireland) is dispensed - 4 replicates - onto the plate, positions D1,2 E1,2.
2.6 200ul of each filtered homogenate is dispensed in duplicate onto the plate, remaining positions.
2.7 50ul of Differential Buffer is dispensed onto the plate bringing the well volume to 250ul.
2.8 The plate is covered with a microtitre plate sealer and incubated at 20°C for 30 minutes.
2.9 The plate is centrifuged for 30 minutes at 2000rpm.
2.10 The plate is then washed 4 times with 150mM PBST.
2.11 50ul of Priming Buffer are added to each well of the microtitre plate and the plate incubated for 1 hour at 37°C.
2.12 The plate is washed 4 times with 150mM PBST.

### 3. Immunoassay:

3.1 250ul of primary prion specific antibody, a rabbit anti-Prion peptide antibody at a dilution of 1:2000, is dispensed onto the plate.
3.2 The plate is incubated at room temperature for 40 minutes.
3.3 The plate is washed 4 times with 150mM PBST.
3.4 250ul of secondary antibody, a donkey-anti-rabbit HRP at a dilution of 1:2000 is dispensed onto the plate and the plate incubated at 37°C for 30 minutes.
3.5 The plate is washed 4 times with 150mM PBST.

### 4. Detection:

4.1 250ul of Enhanced Chemiluminescent reagent (amerlite reagent, supplied by Johnson & Johnson Clinical Diagnostics, U.K.) is added to the plate.
4.2 The plate is incubated at room temperature for 10 minutes.
4.3 The light signal is read using a Labsystems Chemiluminometer (supplied by Medical Supply Co., Dublin, Ireland), which is a scanning wavelength reader. The device reads the luminescence from each well of the plate by scanning the entire IR-visible-UV spectra and extrapolating results.
5.4 The light signals from the plate are transferred to a customised software package (available from G.K.S. Software, Dublin, Ireland)
4.5 Each light signal is assigned to a corresponding barcode and a report printed.
4.6 Results are quoted in chemiluminescent light unit L.U.

### SOURCE OF REAGENTS

1. Plate Adhering Agent, Homogenising Buffer, Priming Buffer and Differential Buffer are supplied by Enfer Products Ltd.
2. Prion Specific Antibodies - anti-PrP - are supplied by Enfer Products and are rabbit antibodies raised to the following synthetic prion peptides. 
PRION SEQUENCE
N-TERMINAL
MVKSHIGSWILVLFVVAMWSDVGLCKKRPKPGGGWNTGGSRYPGQ-44
GSPGGNRYPPQGGGGWGQPHGGGWGQPHGGGWGQPHGGGQGQP-87
GGGGWGQGGSHSQWNKPSKPPKTNMKHVAGAAAGAVVGGLGGY-131
MLGSAMSSPLIHFGNDYEDRYTRENMYRYPNQVYYRPVDRYSNQNN-177
   All the sequences used herein are given using standard I.U.P.A.C. three letter code abbreviations for amino acid residues to find as follows:
   A - Alanine, C - Cysteine, D - Aspartic acid, E - Glutamic acid, F - Phenylalanine, G - Glycine, H - Histidine, I - Isoleucine, K - Lysine, L - Leucine, M - Methionine, N - Asparagine, P - Proline, Q - Glutamine, R - Arginine, S - Serine, T - Threonine, V - Valine, W - Tryptophan and Y - Tyrosine.

   Both underlined sequences, (a 34 amino-acid peptide and a 40 amino acid peptide) are used to raise rabbit anti-PrP antibodies. The peptides are conjugated to activated ovalbumin and injected intra-muscularly in Freund's Complete Adjuvant. Booster injections are sub-cutaneous and Freund's Incomplete Adjuvant is used. The rabbits are bled at 30 days.
3. Chemiluminescent reagent is supplied by Johnson and Johnson, Clinical Diagnostics, U.K. and results are read using a Labsystems Chemiluminometer.

### Example 3

### VALIDATION DATA

### 1. INTER-ASSAY VARIATION

Data generated on a positive control and a negative control, in a total of 10 assays are provided in Table 1. These controls have been deemed positive and negative by two unrelated methods - histology (HIS) and immunohistochemistry (ICC), methods which will ultimately be used to confirm results reported using the Enfer test. The inter-assay variations based on these two samples are 19% for the positive control and 93% for the negative control. These data indicate that the assay has acceptable reproducibility. Another control was also included in this study. This was a peptide control which allows study of the variation between assays due to antibody differences from day to day. These data are included in Table 1. Again the inter-assay variation at 14% is satisfactory.

### 2. INTRA-ASSAY VARIATION

The intra-assay variation was determined using the same type controls as those used for the inter-assay study (positive, negative and peptide) and placing 23 replicates of each control on a single plate. Data generated on these controls are provided in Table 2 showing 9% variation for the positive control, 11% for the peptide control and 57% for the negative control.

### 3. STABILITY STUDIES

A number of stability studies were carried out for the purpose of this validation:

### (a) HOMOGENATE STABILITY

In this case a sample was homogenised and divided into aliquots. Over a seven day period, the same homogenate was tested on four occasions using aliquots stored at -20°C, 2-8°C, and on two occasions using aliquots stored at room temperature and 37°C. The results, outlined in Table 3, show that the light signal is stable, allowing for inter-assay variation, over this period with storage conditions of -20°C, with some deterioration at 2-8°C, room temperature and 37°C.

### (b) STABILITY OF HOMOGENISING BUFFER (HB)

This study was set up to determine the expiry date of homogenising buffer for production purposes. A similar protocol to that outlined in (a) above was followed with the results provided in Table 4. Again the results show that the HB is stable for use.

### (c) STABILITY OF PRIMARY ANTIBODY - WORKING STRENGTH

Undiluted antibody can be stored frozen for an indefinite length of time. However antibody diluted to working strength using PBS/Tween 20 (0.05%) is not as stable as concentrated antibody and a stability study was therefore carried out. Antibody dilutions of 1:1K were made on 9 occasions over a 72 day period. On the final day of the study, each of the 9 preparations was applied to an antigen coated plate and an ELISA carried out. The results are presented in Table 5 and from these data it can be seen that the working strength antibody is stable for at least a 70 day period.

### (d) STABILITY OF SAMPLE IN HOMOGENISING BUFFER BEFORE HOMOGENISATION

This stability test was carried out to ensure that the assay would not be affected if some factor resulted in a delay in homogenising the sample after the HB had been added. Homogenising buffer was added to a sample in the ratio of 1g of brain to 15ml of buffer and the mixture left at room temperature overnight (15 hours). The sample was homogenised after this time and an immunoassay carried out. The results are shown in Table 6 (- 2 samples tested one without and one with a delay). The treatment made no difference to the eventual result.

**Table 1 :**

| *Inter*-*assay variation data*, *generated on a single positive and negative control sample, in 10 assays*. | | | | |
|---|---|---|---|---|
| Assay Number | Blank | 'Neg' Control | 'Pos' Control | Peptide Control |
| 1 | 2 | 4 | 30834 | 110524 |
| day 1 | | | | |
| 2 | 2 | 13 | 33789 | 105242 |
| day 1 | | | | |
| 3 | 2 | 12 | 35319 | 104293 |
| day 1 | | | | |
| 4 | 15 | 23 | 32533 | 101227 |
| day 2 | | | | |
| 5 | 13 | 15 | 35424 | 114084 |
| day 2 | | | | |
| 6 | 17 | 15 | 31749 | 124122 |
| day 3 | | | | |
| 7 | 28 | 50 | 22732 | 146110 |
| day 3 | | | | |
| 8 | 7 | 13 | 29143 | 114355 |
| day 4 | | | | |
| 9 | 18 | 22 | 28565 | 111084 |
| day 4 | | | | |
| 10 | 13 | 12 | 36213 | 100920 |
| day 4 | | | | |
| * The value stated at each data point is the mean value of replicates of each control in the assays. The overall mean stated below is calculated using every individual replicate value. The values are quoted in chemiluminescence light units - LU. | | | | |

| Blank | 'Neg' Control | 'Pos' Control | Peptide Control |
|---|---|---|---|
| Mean = 11 | Mean = 16 | Mean = 31927 | Mean = 111691 |
| SD = 10 | SD = 15 | SD = 5954 | SD = 15331 |
| CV = 91% | CV = 93% | CV = 19% | CV = 14% |
| n = 46 | n = 51 | n = 51 | n = 42 |

**Table 2 :**

| *Intra*-*assay variation data, generated on a single positive and negative control sample, 23 replicates of each on a single plate*. | | | | |
|---|---|---|---|---|
| Replicate Number | Blank | 'Neg' Control | 'Pos' Control | Peptide Control |
| 1 | 34 | 69 | 19857 | 96632 |
| 2 | 49 | 34 | 17738 | 95999 |
| 3 | 22 | 39 | 18354 | 112017 |
| 4 | 29 | 87 | 19645 | 121987 |
| 5 | 38 | 33 | 18975 | 119077 |
| 6 | 51 | 149 | 19828 | 118386 |
| 7 | 34 | 32 | 16466 | 103699 |
| 8 | 38 | 40 | 18259 | 106592 |
| 9 | 24 | 16 | 19840 | 119148 |
| 10 | 32 | 155 | 22813 | 101386 |
| 11 | 26 | 20 | 23004 | 88538 |
| 12 | 17 | 84 | 22153 | 99612 |
| 13 | 57 | 46 | 21880 | 99803 |
| 14 | 32 | 28 | 21722 | 92699 |
| 15 | 25 | 30 | 21069 | 117642 |
| 16 | 57 | 137 | 18524 | 82359 |
| 17 | 18 | 41 | 19644 | 88442 |
| 18 | 45 | 46 | 22478 | 97731 |
| 19 | 15 | 162 | 21596 | 92286 |
| 20 | 20 | 34 | 22438 | 115472 |
| 21 | 13 | 21 | 21661 | 118239 |
| 22 | 33 | 21 | 20113 | 97596 |
| 23 | 19 | 13 | 20007 | 93713 |
| * The values are quoted in chemiluminescence light units - L.U | | | | |

| Blank | 'Neg' Control | 'Pos' Control | Peptide Control |
|---|---|---|---|
| Mean = 32 | Mean = 72 | Mean = 20351 | Mean = 104742 |
| SD = 13 | SD = 41 | SD = 1753 | SD = 11601 |
| CV = 41% | CV = 57% | CV = 9.0% | CV = 11% |
| n = 23 | n = 23 | n = 23 | n = 23 |

**Table 3 :**

| *Homogenate Stability Study - mean of 3 replicates at each temperature* | | | | |
|---|---|---|---|---|
| **POSITIVE CONTROL** | | | | |
| Day | -20°C | 2-8°C | RT | 37°C |
| 0 | | | 1550 | |
| 2 | 1073 | 449 | 664 | 205 |
| 3 | 1162 | 413 | - | - |
| 5 | 2025 | 887 | - | - |
| 7 | 1661 | 1198 | 709 | 472 |
| 30 | | | | |
| 90 | | | | |
| 180 | | | | |

| **NEGATIVE CONTROL** | | | | |
|---|---|---|---|---|
| Day | -20°C | 2-8°C | RT | 37°C |
| 0 | | | 39 | |
| 2 | 10 | 15 | 6 | 6 |
| 3 | 9 | 12 | - | - |
| 5 | 17 | 37 | - | - |
| 7 | 20 | 31 | 10 | 11 |
| 30 | | | | |
| 90 | | | | |
| 180 | | | | |
| * The value at each data point is represented by 3 replicates. | | | | |

**Table 4 :**

| *Homogenising Buffer Stability Study - mean of 3 replicates at each temperature* | | | | |
|---|---|---|---|---|
| **POSITIVE CONTROL** | | | | |
| Day | -20°C | 2-8°C | RT | 37°C |
| 0 | | | 24719 | |
| 2 | 53526 | 61248 | 42873 | 47088 |
| 3 | - | 31593 | 24762 | - |
| 7 | 32420 | 34457 | 36337 | 35010 |
| 30 | | | | |
| 60 | | | | |
| 180 | | | | |

| **NEGATIVE CONTROL** | | | | |
|---|---|---|---|---|
| Day | -20°C | 2-8°C | RT | 37°C |
| 0 | | | 37 | |
| 2 | 4 | 6 | 3 | 4 |
| 3 | - | 9 | 9 | - |
| 7 | 14 | 16 | 20 | 14 |
| 30 | | | | |
| 60 | | | | |
| 180 | | | | |

The value at each data point is represented by 3 replicates.

**Table 5 :**

| *Primary Antibody Stability Study* | | | |
|---|---|---|---|
| Day | 1 °Antibody Mean Light Signal | SD - 8 Replicates | % CV |
| I | 19849 | 557 | 2.8 |
| 3 | 17677 | 77 | 0.4 |
| 4 | 21796 | 340 | 1.6 |
| 9 | 18283 | 218 | 1.2 |
| 23 | 16805 | 282 | 1.7 |
| 25 | 21285 | 377 | 1.8 |
| 30 | 21059 | 427 | 2.0 |
| 42 | 18339 | 353 | 1.9 |
| 72 | 17774 | 511 | 2.9 |

**Table 6 :**

| *Study of sample stability in HB for a 15 hour period before homogenising* | | |
|---|---|---|
| Sample I.D. | Hours before homogenisation | Mean Result (LU) |
| 79 | 0 | 73233 |
| POS | 15 | 72578 |
| | | |
| 80 | 0 | 18 |
| NEG | 15 | 38 |
| * The mean values stated represent 4 replicates at each data point. | | |

## Claims

1. A method "in vitro" for detecting the putative agent for TSE in animals comprising reacting a body tissue sample taken from an animal in a immunological assay with a labelled antibody which is capable of reacting with PrP^{SC} and determining the amount of labelled antibody bound to the sample, **characterized in that** the antibody is a prion specific antibody raised against one or more of the following sequences:

MVKSHIGSWILVLFVVAMWSDVGLCKKRPKPGGGWNTGGSRYPGQ-44
GSPGGNRYPPQGGGGWGQPHGGGWGQPHGGGWGQPHGGGQGQP-87
GGGGWGQGGSHSQWNKPSKPPKTNMKHVAGAAAGAVVGGLGGY-131
MLGSAMSSPLIHFGNDYEDRYTRENMYRYPNQVYYRPVDRYSNQNN-177

2. A method as claimed in claim 1 wherein the antibodies are antibodies raised against the underlined sequences shown in claim 1.

3. A method as claimed in any preceding claim wherein the immunological assay is a competitive assay in which a solid support, suitably a microtitre plate, is pre-coated with a carrier protein-peptide conjugate, the animal tissue sample and an anti-peptide antibody are added to the solid support, allowed to react and the support washed, a labelled anti-(anti-peptide antibody) antibody is added, allowed to react, washed, a signal reagent added and the signal read.

4. The method as claimed in any preceding claim wherein the animals are selected from cattle, sheep and pigs and the tissue sample is taken from a carcass of such animals.

5. A method as claimed in any preceding claim wherein a sample of CNS tissue, suitably a cross-section of spinal cord, is used for testing.

6. A test kit for the detection of TSE in animals comprising an anti-peptide antibody, wherein the antibody is a prion specific antibody raised against one or more of the following sequences:

MVKSHIGSWILVLFVVAMWSDVGLCKKRPKPGGGWNTGGSRYPGQ-44
GSPGGNRYPPQGGGGWGQPHGGGWGQPHGGGWGQPHGGGQGQP-87
GGGGWGQGGSHSQWNKPSKPPKTNMKHVAGAAAGAVVGGLGGY-131
MLGSAMSSPLIHFGNDYEDRYTRENMYRYPNQVYYRPVDRYSNQNN-177

## Patentansprüche

1. Ein "in vitro"-Verfahren zur Detektion des mutmaßlichen Agens für TSE in Tieren, umfassend das Reagieren einer Körpergewebeprobe, die einem Tier entnommen worden ist, in einem immunologischen Assay mit einem markierten Antikörper, der in der Lage ist, mit PrP^{SC} zu reagieren, und das Bestimmen der Menge an markiertem Antikörper, der an die Probe gebunden ist, **dadurch gekennzeichnet, dass** der Antikörper ein Prion-spezifischer Antikörper ist, der gegen eine oder mehrere der folgenden Sequenzen gezüchtet ist:

MVKSHIGSWILVLFVVAMWSDVGLCKKRPKPGGGWNTGGSRYPGQ-44
GSPGGNRYPPQGGGGWGQPHGGGWGQPHGGGWGQPHGGGQGQP-87
GGGGWGQGGSHSQWNKPSKPPKTNMKHVAGAAAGAVVGGLGGY-131
MLGSAMSSPLIHFGNDYEDRYTRENMYRYPNQVYYRPVDRYSNQNN-177

2. Ein Verfahren gemäß Anspruch 1, worin die Antikörper Antikörper sind, die gegen die in Anspruch 1 dargestellten unterstrichenen Sequenzen gezüchtet sind.

3. Ein Verfahren gemäß einem der vorhergehenden Ansprüche, worin der immunologische Assay ein kompetitiver Assay ist, in welchem man einen festen Träger, geeigneterweise eine Mikrotiterplatte, mit einem Carrierprotein-Peptid-Konjugat vorbeschichtet, die Tiergewebeprobe und einen anti-Peptid-Antikörper zu dem festen Träger zugibt, reagieren lässt und den Träger wäscht, einen markierten anti-(anti-Peptid-Antikörper) Antikörper zugibt, reagieren lässt, wäscht, ein Signalreagenz zugibt und das Signal abliest.

4. Das Verfahren gemäß einem der vorhergehenden Ansprüche, worin die Tiere aus Rindern, Schafen und Schweinen ausgewählt sind und die Gewebeprobe aus einer Leiche solcher Tiere entnommen wird.

5. Ein Verfahren gemäß einem der vorhergehenden Ansprüche, worin eine Probe von ZNS-Gewebe, geeigneterweise ein Querschnitt durch das Rückenmark, zum Testen verwendet wird.

6. Ein Test-Kit zur Detektion von TSE in Tieren, umfassend einen anti-Peptid-Antikörper, worin der Antikörper ein Prion-spezifischer Antikörper ist, der gegen eine oder mehrere der folgenden Sequenzen gezüchtet ist:

MVKSHIGSWILVLFVVAMWSDVGLCKKRPKPGGGWNTGGSRYPGQ-44
GSPGGNRYPPQGGGGWGQPHGGGWGQPHGGGWGQPHGGGQGQP-87
GGGGWGQGGSHSQWNKPSKPPKTNMKHVAGAAAGAVVGGLGGY-131
MLGSAMSSPLIHFGNDYEDRYTRENMYRYPNQVYYRPVDRYSNQNN-177

## Revendications

1. Procédé « in vitro » pour la détection de l'agent putatif de la TSE chez les animaux, comprenant la réaction d'un échantillon de tissu corporel prélevé d'un animal dans un essai immunologique avec un anticorps marqué qui est capable de réagir avec PrP^{SC} et la détermination de la quantité d'anticorps marqué lié à l'échantillon, **caractérisé en ce que**
l'anticorps est un anticorps spécifique de prion produit contre une ou plusieurs des séquences suivantes :

MVKSHIGSWILVLFVVAMWSDVGLCKKRPKPGGGWNTGGSRYPGQ-44
GSPGGNRYPPQGGGGWGQPHGGGWGQPHGGGWGQPHGGGQGQP-87
GGGGWGQGGSHSQWNKPSKPPKTNMKHVAGAAAGAVVGGLGGY-131
MLGSAMSSPLIHFGNDYEDRYTRENMYRYPNQVYYRPVDRYSNQNN-177

2. Procédé selon la revendication 1, dans lequel les anticorps sont des anticorps produits contre les séquences soulignées montrées dans la revendication 1.

3. Procédé selon l'une quelconque des revendications précédentes, dans lequel l'essai immunologique est un essai compétitif dans lequel un support solide, de manière adaptée une plaque de microtitration, est pré-revêtu d'un conjugué de protéine-peptide transporteur, l'échantillon de tissu animal et un anticorps anti-peptide sont ajoutés au support solide, laissés réagir et le support lavé, un anticorps marqué anti-(anticorps anti-peptide) est ajouté, laissé réagir, lavé, un réactif de signal ajouté et le signal lu.

4. Procédé selon l'une quelconque des revendications précédentes, dans lequel les animaux sont choisis parmi les bovins, les moutons et les cochons, et l'échantillon de tissu est prélevé d'une carcasse de ces animaux.

5. Procédé selon l'une quelconque des revendications précédentes, dans lequel un échantillon de tissu de SNC, de manière adaptée une section transversale de moelle épinière, est utilisé pour faire le test.

6. Kit de test pour la détection de la TSE chez les animaux, comprenant un anticorps anti-peptide, dans lequel l'anticorps est un anticorps spécifique de prion produit contre une ou plusieurs des séquences suivantes :
